# EUROPÄISCHE PATENTSCHRIFT

(11) **EP 1 235 555 B1**
(45) Veröffentlichungstag und Bekanntmachung des Hinweises auf die Patenterteilung: **19.05.2004**
(21) Anmeldenummer: 00989954.3
(22) Anmeldetag: 06.12.2000
(51) Int. Cl.: A61K 9/00

(54) **STABILE GALENISCHE ZUBEREITUNGEN UMFASSEND EIN BENZIMIDAZOL UND VERFAHREN ZU IHRER HERSTELLUNG**
STABLE GALENIC PREPARATIONS COMPRISING A BENZIMIDAZOL AND METHOD FOR THE PRODUCTION THEREOF
PREPARATIONS GALENIQUES STABLES CONTENANT UN BENZIMIDAZOLE, ET SON PROCEDE DE PRODUCTION

(30) Priorität: 09.12.1999 DE 19959419
(43) Veröffentlichungstag der Anmeldung: 04.09.2002
(73) Patentinhaber: Ratiopharm GmbH, 89070 Ulm (DE)
(72) Erfinder: SCHMITT, Benoit, Irvine, CA 92614 (US)
(74) Vertreter: Best, Michael, Dr.
(86) Internationale Anmeldenummer: PCT/EP2000/012286
(87) Internationale Veröffentlichungsnummer: WO 2001/041734

(56) Entgegenhaltungen:
- EP-A- 0 247 983
- EP-A- 0 773 025

## Beschreibung

Die Erfindung betrifft eine neue galenische Formulierung eines Benzimidazols, insbesondere von Omeprazol oder einer ähnlichen Verbindung, die stabil ist, obwohl sie keine alkalisch reagierende Verbindung enthält, sondern eine sauer reagierende Verbindung in Kontakt mit dem Benzimidazol. Die Erfindung betrifft auch ein Verfahren zur Herstellung einer derartigen Formulierung.

Es wird allgemein angenommen, daß Benzimidazole, wie Omeprazol, in einer sauren Umgebung nicht stabil sind und daß deshalb galenische Formulierungen, die ein derartiges Benzimidazol enthalten, eine alkalisch reagierende Verbindung enthalten sollten. Ein Beispiel für eine derartige Formulierung wird in EP-A 0 247 983 beschrieben.

Durch Kombination der Benzimidazole mit einer ausreichenden Menge alkalisch reagierender Verbindung können galenische Formulierungen erzielt werden, die eine recht gute Stabilität aufweisen. Derartige galenische Formulierungen haben jedoch inhärente Probleme. Z.B. sind in dem enterischen Überzug der Formulierungen saure Gruppen vorhanden, die mit der alkalisch reagierenden Verbindung des Kerns reagieren können. Während es wohlbekannt ist, den alkalisch reagierenden Kern von dem enterischen Überzug durch eine Zwischenschicht zu trennen, muß während der Herstellung derartiger galenischer Formulierungen große Sorgfalt angewandt werden, um die Dicke und Art der Zwischenschicht und die Art des enterischen Überzugs so an die Alkalinität des Kerns anzupassen, daß eine galenische Formulierung mit ausreichender Stabilität und guter Bioverfügbarkeit erhalten wird. Derartige Probleme werden beispielsweise in Beispiel 1 von EP-A 0 247 983 diskutiert.

Vor Kurzem wurden einige Formulierungen entwickelt, bei denen das Benzimidazol nicht mit einer alkalisch reagierenden Verbindung kombiniert wird. Ein Beispiel einer solchen Entwicklung wird in US-A 5,626,875, entsprechend EP-A 0 773 025, beschrieben. In dieser Druckschrift wird eine Formulierung offenbart, umfassend
- einen inerten Kern,
- der mit einer ersten Schicht überzogen ist, die das Benzimidazol zusammen mit einem wasserlöslichen Polymer umfaßt,
- eine weitere Schicht umfassend ein wasserlösliches Polymer und
- einen enterischen Überzug.

In den Beispielen dieser Druckschrift wird das Benzimidazol mit Hilfsstoffen formuliert, die allgemein als inert angesehen werden, wie z.B. Talk. Talk in pharmazeutischer Qualität kann jedoch Verunreinigungen enthalten, und ein pH-Wert von mehr als 7 wird oft in einer Suspension von Talk in Wasser gemessen (ISFET-Technik oder Phenolrotindikator).

Mehrere vor kurzem entwickelte Omeprazol-haltige Arzneimittel kombinieren Omeprazol mit einem spezifischen Stabilisator, um die Stabilität des Mittels zu erhöhen, wie z.B. Mannitol (EP-A 646 006), TiO₂ (WO 96/37195) oder Cyclodextrinen (WO 98/40069) oder Aminosäuren. Es wäre jedoch wünschenswert, ein Omeprazolhaltiges Arzneimittel zur Verfügung zu haben, das ohne einen derartigen Stabilisator ausreichend stabil ist.

Es gibt ein Bedürfnis nach Arzneimitteln, die Benzimidazolverbindungen und insbesondere Omeprazol oder eine ähnliche Verbindung als Wirkstoff enthalten, die eine hervorragende Stabilität kombiniert mit einer guten Bioverfügbarkeit haben und die nicht die Probleme und Nachteile einiger Arzneimittel des Standes der Technik zeigen.

Die vorliegende Erfindung beruht auf dem unerwarteten Befund, daß die Benzimidazole, die für sehr säurelabil gehalten wurden, tatsächlich eine hervorragende Stabilität aufweisen, wenn sie zusammen mit einer Verbindung formuliert werden, die einen pH-Wert von weniger als 7 zur Verfügung stellt, wenn sie in Kontakt mit ausreichend Wasser gebracht wird, um einen pH-Wert zu messen (sauer reagierende Verbindung, saurer Stoff).

Die Erfindung stellt somit ein stabiles orales Arzneimittel zur Verfügung, das eine Benzimidazolverbindung der Formel I 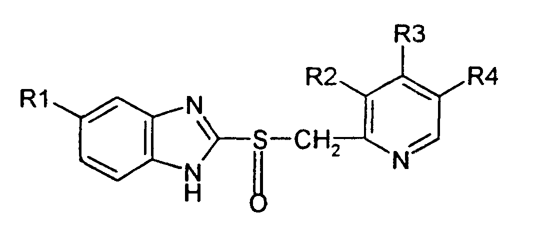 enthält, worin R1 Wasserstoff, Methoxy, oder Difluormethoxy ist, R2 Wasserstoff, Methyl oder Methoxy ist, R3 Methoxy, 2,2,2-Trifluorethoxy oder 3-Methoxypropoxy ist und R4 Wasserstoff, Methyl oder Methoxy ist, umfassend:
(a) einen inerten Kern,
(b) darauf eine Wirkstoffschicht, die die Benzimidazolverbindung der Formel I im Gemisch mit einer sauer reagierenden Verbindung und gegebenenfalls pharmazeutisch verträglichen Hilfsmitteln enthält,
(c) wenigstens eine inerte Schicht und
(d) eine äußere Schicht, die auf der inerten Schicht aufgebracht ist und die einen enterischen Überzug umfaßt.

Die Erfindung stellt auch ein Verfahren zur Herstellung der erfindungsgemäßen Arzneimittel bereit, das die folgenden Schritte umfaßt:
(i) Bereitstellung eines inerten Kerns
(ii) Überziehen des inerten Kerns mit einer Benzimidazolschicht,
(iii) Aufbringen einer oder mehrerer inerter Schichten
(iv) Aufbringen des enterischen Überzugs.

Die Benzimidazole der Formel I sind allgemein als wirksame Inhibitoren der Magensäuresekretion bekannt. Typische Beispiele solcher Benzimidazolverbindungen sind Omeprazol, Lanzoprazol und Pantoprazol. Am meisten bevorzugt ist Omeprazol.

Der inerte Kern ist vorzugsweise ein bekannter Zucker-/Stärke-Kern. Dieser inerte Kern ist mit der Benzimidazolschicht überzogen. Die Benzimidazolschicht enthält den Wirkstoff, das Benzimidazol, in Kombination mit der sauer reagierenden Verbindung. Pharmazeutisch verträgliche Hilfsstoffe können ebenfalls vorhanden sein. Im allgemeinen wird die Benzimidazolschicht ein wasserlösliches Polymer (das bevorzugt nicht-alkalisch ist) als Bindemittel enthalten, was nachstehend erläutert wird.

Die Benzimidazolschicht ist mit dem inerten Überzug überzogen, der den ersten Überzug, der das Benzimidazol enthält, und den enterischen Überzug trennt. Der inerte Überzug enthält im allgemeinen ein wasserlösliches Polymer und gegebenenfalls andere pharmazeutisch verträgliche Hilfsstoffe.

Es ist bevorzugt, daß der pH-Wert einer wäßrigen Suspension oder Lösung, die von dem inerten Überzug erhalten wird, etwa 7 ist. Wenn zwei inerte Überzüge vorhanden sind, ist der pH-Wert einer wäßrigen Suspension oder Lösung des ersten inerten Überzugs vorzugsweise niedriger als der pH-Wert einer wäßrigen Suspension oder Lösung des zweiten inerten Überzugs.

Es ist bevorzugt, daß wenigstens zwei inerte Überzüge (oder Schichten; "Schicht" und "Überzug" wird im Rahmen dieser Beschreibung synonoym verwendet) vorhanden sind. Der erste inerte Überzug umfaßt im allgemeinen ein wasserlösliches Polymer, das vorzugsweise nicht-alkalisch ist, gegebenenfalls eine sauer reagierende Verbindung und gegebenenfalls pharmazeutisch verträgliche Hilfsstoffe. Der zweite inerte Überzug, der zwischen dem ersten inerten Überzug und dem enterischen Überzug aufgebracht ist, umfaßt im allgemeinen ein wasserlösliches Polymer, das vorzugsweise nicht-alkalisch ist, gegebenenfalls eine sauer reagierende Verbindung und gegebenenfalls pharmazeutisch verträgliche Hilfsstoffe. Der zweite inerte Überzug und der erste inerte Überzug haben vorzugsweise unterschiedliche Zusammensetzungen.

Der erste inerte Überzug (falls vorhanden) dient als Barriere zwischen der wirksamen Benzimidazolschicht und dem zweiten inerten Überzug, der Bestandteile enthalten kann, die nicht in Kontakt mit der Benzimidazolschicht kommen sollten. Diese erste Schicht enthält normalerweise ein wasserlösliches Polymer, vorzugsweise eine sauer reagierende Verbindung und übliche Hilfsstoffe, wie z.B. ein Gleitmittel. Als Gleitmittel ist Polyethylenpolypropylenglycol, bekannt als Poloxamer, bevorzugt.

Die zweite inerte Schicht dient als Zwischenschicht zwischen dem enterischen Überzug und dem inneren Teil der Zubereitung. Sie umfaßt normalerweise ein wasserlösliches Polymer und ist üblicherweise frei von der sauer reagierenden Verbindung (kann diese aber umfassen). Sie kann weiterhin übliche Hilfsstoffe wie Talk und Pigmente, z.B. Titandioxid, umfassen.

Wenn nur eine inerte Schicht vorhanden ist, hat diese inerte Schicht üblicherweise die gleiche Zusammensetzung wie die vorstehend beschriebene zweite inerte Schicht.

Die enterische Beschichtung dient als klassischer enterischer Überzug. Sie umfaßt übliche enterische Substanzen, die im Stand der Technik bekannt sind, wie Cellulosephthalate und Copolymere vom Methacrylsäure-Typ, z.B.

Methacrylsäure/(Meth)acrylsäurealkylester-Copolymere, die beispielsweise unter der Marke Eudragit® verkauft werden. Als Alkylrest sind ein Methylrest und ein Ethylrest bevorzugt.

Bevorzugt ist das Methacrylsäure-Typ C-Copolymer nach der US-Pharmacopeia. Besonders bevorzugt ist das Produkt Eudragit L30D55. Ein bevorzugtes Polymer ist ein Copolymer auf der Basis von Methacrylsäure und Acrylsäureethylester. Die Formel ist wie folgt:

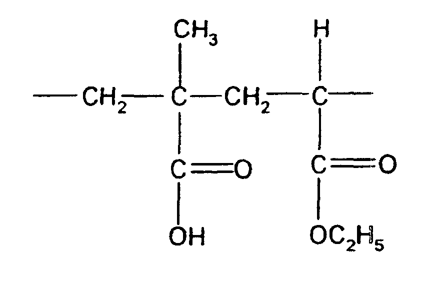

Das Verhältnis freier Carboxylgruppen zu Estergruppen ist vorzugsweise etwa 1:1. Das durchschnittliche Molekulargewicht ist beispielsweise etwa 250.000.

Ein derartiges Copolymer wird sich bei pH-Werten oberhalb 5,5 unter Salzbildung leicht lösen.

Triethylcitrat und/oder Polyethylenglycol oder ähnliche Verbindungen können in dem enterischen Überzug als Weichmacher verwendet werden. Talk kann auch vorhanden sein. Andere übliche Hilfsstoffe können auch vorhanden sein.

Die sauer reagierende Verbindung, die in den Arzneimitteln der vorliegenden Erfindung enthalten ist, ist nicht besonders eingeschränkt. Die sauer reagierende Verbindung ist dadurch definiert, daß sie einen pH-Wert von weniger als 7 liefert, wenn sie in Wasser suspendiert oder gelöst wird.

Insbesondere sollte das zum Beschichten verwendete wäßrige Gemisch, das das Benzimidazol und die sauer reagierende Verbindung enthält, einen pH-Wert von weniger als 7 aufweisen, vorzugsweise einen pH-Wert in dem Bereich von 6,5 bis 6,95, stärker bevorzugt in dem Bereich von 6,6 bis 6,95.

Entsprechend sollte die Schicht, die Omeprazol und die sauer reagierende Verbindung enthält, wenn sie in Wasser suspendiert wird, einen pH-Wert von weniger als 7 liefern. Vorzugsweise sollte eine Menge von 5 bis 50 mg des Schichtmaterials suspendiert in 100 mg Wasser einen pH-Wert im Bereich von 6,5 bis 6,95, vorzugsweise von 6,6 bis 6,95 liefern. Die pH-Messungen sollten vorzugsweise unmittelbar nach dem Suspendieren des Schichtmaterials in Wasser erfolgen. "Unmittelbar" bedeutet in diesem Zusammenhang innerhalb von 30 Minuten, vorzugsweise innerhalb von 5 Minuten, nachdem das Material in das Wasser gegeben wurde. Aus praktischen Gründen kann es vorteilhaft sein, den pH-Wert in einer Lösung oder Suspension zu messen, die den inerten Kern zusammen mit der Benzimidazol-haltigen Schicht enthält. Wenn die Messung in Gegenwart des inerten Kerns durchgeführt wird, ist es besonders wichtig, daß die Messung unmittelbar (vorzugsweise sobald wie möglich) durchgeführt wird, nachdem das Material in das Wasser gebracht wurde, um das Risiko zu vermeiden, daß Kernmaterial sich löst und den pH-Wert beeinflußt. Insbesondere sollte der Kern nicht zerkleinert werden, so daß sich das Kernmaterial so langsam wie möglich auflöst.

Wenn der inerte Überzug die sauer reagierende Verbindung enthält, sollte der pH-Wert einer Lösung oder Suspension des inerten Überzugs weniger als 7 sein, vorzugsweise sollten 5 bis 50 mg des Überzugsmaterials suspendiert oder gelöst in 100 mg Wasser einen pH-Wert im Bereich von 6,5 bis 6,95, stärker bevorzugt im Bereich von 6,6 bis 6,95 liefern.

Wie der pH-Wert gemessen wird, ist nicht entscheidend, bevorzugt ist die bekannte ISFET-Technologie unter Verwendung einer ISFET-Elektrode. Falls nicht anders angegeben, wurden pH-Messungen, auf die in dieser Beschreibung Bezug genommen wird, mit der ISFET-Technologie bei einer Temperatur von 25°C gemacht.

Die sauer reagierende Verbindung ist vorzugsweise Natriumdihydrogenphosphat (NaH₂PO₄), mit dem die besten Ergebnisse bezüglich der Stabilität erreicht werden können. Die Menge der sauer reagierenden Verbindung wird durch den gewünschten pH-Wert bestimmt.

Die sauer reagierende Verbindung ist notwendigerweise in der Schicht vorhanden, die das Benzimidazol enthält und vorzugsweise vorhanden in der inerten Schicht. Wenn zwei inerte Schichten vorhanden sind (bevorzugte Ausführungsform), ist die sauer reagierende Verbindung vorzugsweise in der ersten inerten Schicht vorhanden, während sie vorzugsweise in der zweiten inerten Schicht nicht vorhanden ist.

Das wasserlösliche Polymer, das im allgemeinen in der Benzimidazolschicht und in der/den inerten Schicht(en) vorhanden ist, wirkt als ein Bindemittel und kann jedes Polymer sein von dem bekannt ist, daß es wasserlöslich ist oder in Wasser rasch zerfällt. Es ist möglich, das gleiche wasserlösliche Polymer in allen Schichten zu verwenden, in denen es vorhanden ist, oder verschiedene wasserlösliche Polymere in verschiedenen Schichten zu verwenden. Bevorzugte wasserlösliche Polymere sind nicht-alkalische wasserlösliche Polymere und besonders bevorzugt sind Hydroxypropylmethylcellulose und Hydroxypropylcellulose.

Übliche pharmazeutisch verträgliche Hilfsstoffe, die einem Fachmann bekannt sind, können in jeder der Schichten des Arzneimittels vorhanden sein. Vorzugsweise liefern diese Hilfsstoffe in wäßriger Lösung oder Suspension einen pH-Wert von etwa 7 (z.B. 5 bis 9). Die Art und Menge dieser Hilfsstoffe kann leicht von einem Fachmann aufgrund seines allgemeinen Fachwissens bestimmt werden.

Wenn nicht anders angegeben, umfassen die Hilfsstoffe übliche Bindemittel, Weichmacher, Farbstoffe, Pigmente wie Titandioxid, Talk und andere bekannte Hilfsstoffe.

Mit der vorliegenden Erfindung ist es nicht notwendig, daß die Benzimidazol-haltige Schicht einen spezifischen Stabilisator für das Benzimidazol enthält, wie es für mehrere Arzneimittel des Standes der Technik beschrieben wird, wie Mannit (EP-A 646 006), TiO₂ (WO 96/37195) oder Cyclodextrine (WO 98/40069) oder Aminosäuren. Vielmehr enthält die Benzimidazol-haltige Schicht vorzugsweise nur das Benzimidazol, das Bindemittel und die sauer reagierende Verbindung.

Das besonders bevorzugte Arzneimittel der vorliegenden Erfindung ist eine Pellet-Formulierung umfassend
(a) einen inerten Kern, der ein Zucker-/Stärke-Kern ist,
(b) überzogen mit einem Gemisch aus Omeprazol, Hydroxypropylmethylcellulose und so viel Natriumdihydrogenphosphat, daß 5 bis 50 mg des Überzugsmaterials in 100 mg Wasser suspendiert oder gelöst einen pH-Wert im Bereich von 6,5 bis 6,95, vorzugsweise 6,6 bis 6,95 liefern,
(c) einen ersten inerten Überzug, aufgebracht auf der Omeprazol-haltigen Schicht, wobei der erste inerte Überzug im wesentlichen aus Hydroxypropylmethylcellulose, Polyethylenpolypropylenglycol und so viel Natriumdihydrogenphosphat besteht, daß 5 bis 50 mg Überzugsmaterial in 100 mg Wasser einen pH-Wert im Bereich von 6,5 bis 6,95, vorzugsweise im Bereich von 6,6 bis 6,95, liefern,
(d) einen zweiten inerten Überzug, aufgebracht auf dem ersten inerten Überzug, wobei der zweite inerte Überzug im wesentlichen aus Hydroxypropylmethylcellulose, Talk und Titandioxid besteht und
(e) eine äußere Schicht aufgebracht auf dem zweiten inerten Überzug, bestehend im wesentlichen aus Methacrylsäure/(Meth)acrylsäurealkylester-Copolymer, Triethylcitrat und Talk.

Es ist ebenfalls bevorzugt, daß ein Pellet, das nur den inerten Kern und den das Benzimidazol enthaltenden Überzug aufweist, einen pH-Wert im Bereich von 6,6 bis 6,95 liefert, wenn es in 4 µl deionisiertem Wasser suspendiert wird. Wenn zwei oder mehr inerte Schichten vorhanden sind, ist es ebenfalls bevorzugt, daß ein Pellet, das nur den inerten Kern, den das Benzimidazol enthaltenden Überzug und den ersten inerten Überzug aufweist, einen pH-Wert im Bereich von 6,6 bis 6,95 liefert, wenn es in 4 µl deionisiertem Wasser suspendiert wird. Wiederum ist es bevorzugt, daß die pH-Messung unmittelbar durchgeführt wird, nachdem das Material zu dem Wasser gegeben wurde, um einen möglichen Einfluß des inerten Kerns zu minimieren (innerhalb von 30 Minuten, vorzugsweise innerhalb von 5 Minuten, nachdem das Material in das Wasser eingebracht wurde).

Die Arzneimittel der vorliegenden Erfindung können durch bekannte Verfahren hergestellt werden. Die Überzugsschritte werden vorzugsweise in einer Fließbettbeschichtungsvorrichtung ausgeführt. Die Arzneimittel der vorliegenden Erfindung liegen in Form von Pellets vor, und die letztendliche Dosierungsform, die dem Patienten verabreicht wird, ist eine Kapsel enthaltend die Pellets. Alternativ ist es auch möglich, die Pellets in eine Tablette zu pressen.

Es ist bevorzugt, daß zwischen den Beschichtungsschritten Trocknungsschritte durchgeführt werden, dies ist allerdings nicht absolut notwendig. Wenn ein Trocknungsschritt durchgeführt wird, ist es nicht notwendig, das Fließbettverfahren zu unterbrechen (das heißt der Trocknungsschritt kann in dem Fließbett durchgeführt werden), aber selbstverständlich muß das Sprühen während des Trocknungsschritts angehalten werden. Wenn ein Trocknungsschritt durchgeführt wird, dauert er etwa 10 bis 20 Minuten.

Die folgenden Beispiele erläutern die Erfindung, sind aber nicht einschränkend.

### Beispiel 1

Eine Dispersion wird hergestellt aus 140 g deionisiertem Wasser, 20 g Omeprazol und 16 g Hydroxypropylmethylcellulose. Der pH-Wert dieser Dispersion wird mit einer wäßrigen 0,5 M Natriumdihydrogenphosphatlösung auf einen pH-Wert von 6,65 eingestellt. Der pH-Wert wird durch eine ISFET-Elektrode gemessen. 140 g Neutral-Pellets werden mit der so hergestellten Suspension in einer Fließbettbeschichtungsvorrichtung überzogen.

Eine Suspension für den inerten Überzug wird durch Dispergieren von 125 g deionisiertem Wasser, 3 g Titandioxid, 5 g Talk und 15 g Hydroxypropylmethylcellulose hergestellt. Die Pellets mit dem Omeprazolüberzug werden mit dieser Suspension in einer Fließbettbeschichtungsvorrichtung überzogen.

Die so hergestellten Pellets werden mit einem enterischen Überzug in bekannter Weise überzogen. Die Suspension des enterischen Überzugs wird aus 30 g Methacrylsäure-Copolymer Typ C (nach US-Pharmacopeia), 12 g Talk, 4,5 g Triethylcitrat und 95,0 g deionisiertem Wasser hergestellt. Wiederum wird eine Fließbettbeschichtungsvorrichtung verwendet.

Die so erhaltenen Pellets sind stabil und stellen eine hervorragende Bioverfügbarkeit bereit.

### Beispiel 2

Beispiel 1 wurde wiederholt, außer daß nach dem Beschichten der inerten Kerne mit der Omeprazol-haltigen Schicht zunächst eine erste Überzugsschicht aufgebracht wurde. Hierzu wurde eine Überzugslösung verwendet, die hergestellt wurde aus 5 g Hydroxypropylmethylcellulose, 1,0 g Poloxamer 188, 40 g deionisiertem Wasser und wäßriger 0,5 M Natriumdihydrogenphosphatlösung, zur Einstellung des pH-Werts auf etwa 6,9. Der Überzug wird in einer Fließbettbeschichtungsvorrichtung aufgebracht. Auf diesen ersten inerten Überzug werden dann der nächste inerte Überzug (zweiter inerter Überzug) und der enterische Überzug aufgebracht, wobei dieselben Komponenten, Mengen und Techniken verwendet werden, wie in Beispiel 1 vorstehend beschrieben.

Die so erhaltenen Pellets zeigen eine überlegene Stabilität und hervorragende Bioverfügbarkeit.

Wenn im Rahmen dieser Anmeldung davon gesprochen wird, daß (eine Menge von) 5 bis 50 mg eines Überzugs- oder Schichtmaterials in 100 mg Wasser einen pH-Wert in einem bestimmten Bereich zur Verfügung stellt, bedeutet dies selbstverständlich, daß es zur Erfüllung des Merkmals ausreichend ist, daß irgendeine Menge an Material aus dem Bereich von 5 bis 50 mg in 100 mg Wasser einen pH-Wert in dem bestimmten Bereich zur Verfügung stellt. Zur Erfüllung dieses Merkmals ist es also beispielsweise ausreichend, wenn 5 mg Schichtmaterial in 100 mg Wasser einen pH-Wert in dem bestimmten Bereich zur Verfügung stellt, auch wenn eine andere Menge Schichtmaterial, wie 10 mg Schichtmaterial (oder 50 mg Schichtmaterial) in 100 mg Wasser einen pH-Wert zur Verfügung stellt, der außerhalb des bestimmten Bereichs liegt.

## Patentansprüche

1. Orales Arzneimittel in Form von Pellets enthaltend eine Benzimidazolverbindung der Formel I 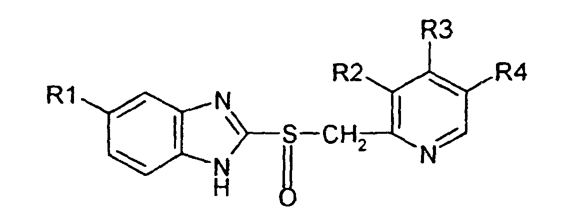 worin R1 Wasserstoff, Methoxy oder Difluormethoxy ist, R2 Wasserstoff, Methyl oder Methoxy ist, R3 Methoxy, 2,2,2-Trifluorethoxy oder 3-Methoxypropoxy ist und R4 Wasserstoff, Methyl oder Methoxy ist, umfassend
(a) einen inerten Kern
(b) darauf eine Wirkstoffschicht, die die Benzimidazolverbindung der Formel I im Gemisch mit einer sauer reagierenden Verbindung und gegebenenfalls pharmazeutisch verträglichen Hilfsmitteln enthält
(c) wenigstens eine inerte Schicht und
(d) eine äußere Schicht, die auf der inerten Schicht aufgebracht ist und die einen enterischen Überzug umfaßt.

2. Arzneimittel nach Anspruch 1, worin eine erste inerte Schicht und eine zweite inerte Schicht vorhanden sind, wobei die erste inerte Schicht auf der Wirkstoffschicht und die zweite inerte Schicht auf der ersten inerten Schicht aufgebracht ist.

3. Arzneimittel nach Anspruch 2, worin die erste inerte Schicht ein inertes wasserlösliches Polymer, gegebenenfalls eine sauer reagierende Verbindung und gegebenenfalls pharmazeutisch verträgliche Hilfsstoffe umfaßt, und die zweite inerte Schicht ein wasserlösliches Polymer und gegebenenfalls pharmazeutisch verträgliche Hilfsstoffe umfaßt.

4. Arzneimittel nach Anspruch 3, in der die erste inerte Schicht eine sauer reagierende Verbindung umfaßt.

5. Arzneimittel nach einem der Ansprüche 1 bis 4, in der das wasserlösliche Polymer Hydroxypropylmethylcellulose und/oder Hydroxypropylcellulose umfaßt.

6. Arzneimittel nach einem der Ansprüche 1 bis 5, in der eine Suspension aus dem inerten Kern und der Wirkstoffschicht eines Pellets in 4 µl Wasser einen pH-Wert im Bereich von 6,5 bis 6,95, insbesondere von 6,6 bis 6,95, aufweist.

7. Arzneimittel nach einem der Ansprüche 1 bis 6, in der die sauer reagierende Verbindung Natriumdihydrogenphosphat umfaßt.

8. Arzneimittel nach einem der Ansprüche 1 bis 7, in der die Wirkstoffschicht und, falls vorhanden, die erste inerte Schicht frei von Talk ist.

9. Arzneimittel nach einem der Ansprüche 1 bis 8, in der die Benzimidazolverbindung der Formel I Omeprazol ist.

10. Arzneimittel nach Anspruch 1, umfassend
(a) einen inerten Kern
(b) darauf eine Wirkstoffschicht, die im wesentlichen aus Omeprazol, Hydroxypropylmethylcellulose und einer sauer reagierenden Verbindung besteht, wobei die sauer reagierende Verbindung in einer Menge vorhanden ist, so daß eine Suspension des inerten Kerns mit der Wirkstoffschicht in 4 µl Wasser einen pH-Wert im Bereich von 6,6 bis 6,95 aufweist,
(c) eine erste inerte Schicht bestehend im wesentlichen aus Hydroxypropylmethylcellulose, einer sauer reagierenden Verbindung und einem Gleitmittel,
(d) eine zweite inerte Schicht bestehend im wesentlichen aus Hydroxypropylmethylcellulose, Talk und einem Pigment, insbesondere TiO₂, und
(e) eine äußere Schicht bestehend im wesentlichen aus einem enterischen Überzug, gebildet aus Methacrylsäure/(Meth)acrylsäurealkylester-Copolymer, Triethylcitrat und Talk.

11. Arzneimittel nach Anspruch 10, worin die sauer reagierende Verbindung Natriumdihydrogenphosphat ist.

12. Verfahren zur Herstellung eines Arzneimittels nach einem der Ansprüche 1 bis 11, das folgende Schritte umfaßt:
(i) Bereitstellen eines inerten Kerns,
(ii) Überziehen des inerten Kerns mit einer Suspension, die die Benzimidazolverbindung der Formel I und zumindest eine sauer reagierende Verbindung enthält und einen pH-Wert im Bereich von 6,5 bis 6,95, insbesondere von 6,6 bis 6,95, aufweist,
(iii) Aufbringen wenigstens einer inerten Schicht,
(iv) Aufbringen einer enterischen Schicht.

13. Verfahren nach Anspruch 12, das die folgenden Schritte umfaßt:
(i) Bereitstellen eines inerten Kerns,
(ii) Überziehen des inerten Kerns mit einer Suspension, die die Benzimidazolverbindung der Formel I und zumindest eine sauer reagierende Verbindung enthält und einen pH-Wert im Bereich von 6,5 bis 6,95, insbesondere von 6,6 bis 6,95, aufweist,
(iii) Aufbringen einer ersten inerten Schicht,
(iv) Aufbringen einer zweiten inerten Schicht und
(v) Aufbringen der enterischen Schicht.

14. Verfahren nach Anspruch 13, wobei die erste inerte Schicht aus einer Überzugslösung oder -suspension aufgebracht wird, die einen pH-Wert im Bereich von 6,5 bis 6,95, insbesondere im Bereich von 6,6 bis 6,95, aufweist.

15. Verfahren nach einem der Ansprüche 1 bis 14, wobei die Schichten mittels einer Fließbettbeschichtungsvorrichtung aufgebracht werden.

## Claims

1. An oral pharmaceutical in the form of pellets comprising a benzimidazole compound of the formula I 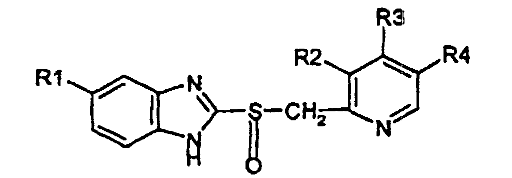 in which R1 is hydrogen, methoxy or difluoromethoxy, R2 is hydrogen, methyl or methoxy, R3 is methoxy, 2,2,2-trifluoroethoxy or 3-methoxypropoxy, and R4 is hydrogen, methyl or methoxy, comprising
(a) an inert core,
(b) thereon an active ingredient layer which comprises the benzimidazole compound of the formula I mixed with an acidic reacting compound and, where appropriate, pharmaceutically acceptable adjuvants,
(c) at least one inert layer and
(d) an outer layer which is applied to the inert layer and which comprises an enteric coating.

2. A pharmaceutical as claimed in claim 1, in which a first inert layer and a second inert layer are present, where the first inert layer is applied to the active ingredient layer, and the second inert layer is applied to the first inert layer.

3. A pharmaceutical as claimed in claim 2, in which the first inert layer comprises an inert water-soluble polymer, where appropriate an acidic reacting compound and, where appropriate, pharmaceutically acceptable excipients, and the second inert layer comprises a water-soluble polymer and, where appropriate, pharmaceutically acceptable excipients.

4. A pharmaceutical as claimed in claim 3, in which the first inert layer comprises an acidic reacting compound.

5. A pharmaceutical as claimed in any of claims 1 to 4, in which the water-soluble polymer comprises hydroxypropylmethylcellulose and/or hydroxypropylcellulose.

6. A pharmaceutical as claimed in any of claims 1 to 5, in which a suspension of the inert core and the active ingredient layer of a pellet in 4 µl of water has a pH in the range from 6.5 to 6.95, in particular from 6.6 to 6.95.

7. A pharmaceutical as claimed in any of claims 1 to 6, in which the acidic reacting compound comprises sodium dihydrogen phosphate.

8. A pharmaceutical as claimed in any of claims 1 to 7, in which the active ingredient layer and, if present, the first inert layer is free of talc.

9. A pharmaceutical as claimed in any of claims 1 to 8, in which the benzimidazole compound of the formula I is omeprazole.

10. A pharmaceutical as claimed in claim 1, comprising
(a) an inert core
(b) thereon an active ingredient layer which consists essentially of omeprazole, hydroxypropylmethylcellulose and an acidic reacting compound, where the acidic reacting compound is present in an amount such that a suspension of the inert core with the active ingredient layer in 4 µl of water has a pH in the range from 6.6 to 6.95,
(c) a first inert layer consisting essentially of hydroxypropylmethylcellulose, an acidic reacting compound and a lubricant,
(d) a second inert layer consisting essentially of hydroxypropylmethylcellulose, talc and a pigment, in particular TiO₂, and
(e) an outer layer consisting essentially of an enteric coating formed from methacrylic acid/alkyl (meth)acrylate copolymer, triethyl citrate and talc.

11. A pharmaceutical as claimed in claim 10, in which the acidic reacting compound is sodium dihydrogen phosphate.

12. A process for producing a pharmaceutical as claimed in any of claims 1 to 11, which comprises the following steps:
(i) preparation of an inert core,
(ii) coating of the inert core with a suspension which comprises the benzimidazole compound of the formula I and at least one acidic reacting compound and has a pH in the range from 6.5 to 6.95, in particular from 6.6 to 6.95,
(iii) application of at least one inert layer,
(iv) application of an enteric layer.

13. The process as claimed in claim 12, which comprises the following steps:
(i) preparation of an inert core,
(ii) coating of the inert core with a suspension which comprises the benzimidazole compound of the formula I and at least one acidic reacting compound and has a pH in the range from 6.5 to 6.95, in particular from 6.6 to 6.95,
(iii) application of a first inert layer,
(iv) application of a second inert layer and
(v) application of the enteric layer.

14. The process as claimed in claim 13, where the first inert layer is applied from a coating solution or suspension which has a pH in the range from 6.5 to 6.95, in particular in the range from 6.6 to 6.95.

15. The process as claimed in any of claims 1 to 14, where the layers are applied by means of a fluidized bed coating apparatus.

## Revendications

1. Médicament oral sous forme de pellets contenant un composé de benzimidazole de formule I 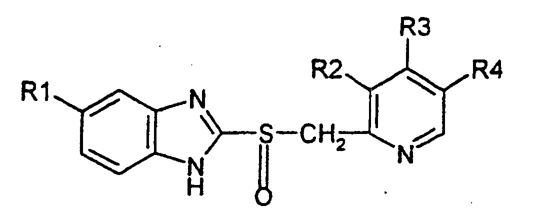 dans laquelle R¹ représente de l'hydrogène ou un radical méthoxy ou difluorométhoxy, R² de l'hydrogène ou un radical méthyle ou méthoxy, R³ un radical méthoxy, 2,2,2-trifluoroéthoxy ou 3-méthoxypropoxy et R⁴ de l'hydrogène ou un radical méthyle ou méthoxy, comprenant:
(a) un noyau inerte,
(b) sur celui-ci, une couche d'ingrédient actif, qui contient le composé de benzimidazole de formule I en mélange avec un composé à réaction acide et éventuellement des adjuvants pharmaceutiquement compatibles,
(c) au moins une couche inerte et
(d) une couche extérieure, qui est appliquée sur la couche inerte et comprend un revêtement entérique.

2. Médicament selon la revendication 1, dans lequel une première couche inerte et une deuxième couche inerte sont présentes, la première couche inerte étant appliquée sur la couche d'ingrédient actif et la deuxième couche inerte sur la première couche inerte.

3. Médicament salon la revendication 2, dans lequel la première couche inerte comprend un polymère inerte soluble dans l'eau, éventuellement un composé à réaction acide et éventuellement des adjuvants pharmaceutiquement compatibles, et la deuxième couche inerte comprend un polymère soluble dans l'eau et éventuellement des adjuvants pharmaceutiquement compatibles.

4. Médicament selon la revendication 3, dans lequel la première couche inerte comprend un composé à réaction acide.

5. Médicament selon l'une quelconque des revendications 1 à 4, dans lequel le polymère soluble dans l'eau comprend de l'hydroxypropylméthylcellulose et/ou de l'hydroxypropylcellulose.

6. Médicament selon l'une quelconque des revendications 1 à 5, dans lequel une suspension du noyau inerte et de la couche d'ingrédient actif d'un pellet dans 4 µl d'eau présente un pH de l'ordre de 6,5 à 6,95, en particulier de 6,6 à 6,95.

7. Médicament selon l'une quelconque des revendications 1 à 6, dans lequel le composé à réaction acide comprend du dihydrogénophosphate de sodium.

8. Médicament selon l'une quelconque des revendications 1 à 7, dans lequel la couche d'ingrédient actif et, si elle est présente, la première couche inerte, sont exemptes de talc.

9. Médicament selon l'une quelconque des revendications 1 à 8, dans lequel le composé de benzimidazole de formule I est de l'Omeprazol.

10. Médicament selon la revendication 1, comprenant
(a) un noyau inerte,
(b) sur celui-ci, une couche d'ingrédient actif, essentiellement constituée d'Omeprazol, d'hydroxypropylméthylcellulose et d'un composé à réaction acide, le composé à réaction acide étant présent en une quantité telle qu'une suspension du noyau inerte avec la couche d'ingrédient actif dans 4 µl d'eau présente un pH de l'ordre de 6,6 à 6,95,
(c) une première couche inerte essentiellement constituée d'hydroxypropylméthylcellulose, d'un composé à réaction acide et d'un lubrifiant,
(d) une deuxième couche inerte essentiellement constituée d'hydroxypropylméthylcellulose, de talc et d'un pigment, en particulier TiO₂, et
(e) une couche extérieure, essentiellement constituée d'un revêtement entérique à base de copolymère d'acide méthacrylique / alkylester d'acide (méth)acrylique, de citrate de triéthyle et de talc.

11. Médicament selon la revendication 10, dans lequel le composé à réaction acide est du dihydrogénophosphate de sodium.

12. Procédé de préparation d'un médicament selon l'une quelconque des revendications 1 à 11, comprenant les étapes suivantes:
(i) fourniture d'un noyau inerte,
(ii) revêtement du noyau inerte par une suspension contenant le composé de benzimidazole de formule I et au moins un composé à réaction acide et présentant un pH de l'ordre de 6,5 à 6,95, en particulier de 6,6 à 6,95,
(v) application d'au moins une couche inerte,
(vi) application d'un revêtement entérique.

13. Procédé selon la revendication 12, comprenant les étapes suivantes:
(i) fourniture d'un noyau inerte,
(ii) revêtement du noyau inerte par une suspension contenant le composé de benzimidazole de formule I et au moins un composé à réaction acide et présentant un pH de l'ordre de 6,5 à 6,95, en particulier de 6,6 à 6,95,
(iii) application d'au moins une première couche inerte,
(iv) application d'une deuxième couche inerte et
(v) application du revêtement entérique.

14. Procédé selon la revendication 13, où la première couche inerte est appliquée à partir d'une solution ou d'une suspension de revêtement, qui présente un pH de l'ordre de 6,5 à 6,95, en particulier de l'ordre de 6,6 à 6,95.

15. Procédé selon l'une quelconque des revendications 1 à 14, où les revêtements sont appliqués au moyen d'un dispositif de revêtement à lit fluide.
